# EUROPEAN PATENT APPLICATION

(11) **EP 4 316 440 A1**
(43) Date of publication of application: **07.02.2024**
(21) Application number: 21935148.3
(22) Date of filing: 25.11.2021
(51) Int. Cl.: A61F 13/47, A61F 13/475, A61F 13/476

(54) **ABSORBENT ARTICLE**

(30) Priority: 30.03.2021 JP 2021057751
(71) Applicant: DAIO PAPER CORPORATION, Shikokuchuo-shi Ehime 799-0492 (JP)
(72) Inventor: YOSHIOKA, Chisaki, Sakura-shi, Tochigi 329-1411 (JP)
(74) Representative: Epping - Hermann - Fischer
(86) International application number: PCT/JP2021/043294
(87) International publication number: WO 2022/208995

(57) **Abstract**

An absorbent article including a main body having a permeable top sheet on an upper side, an impermeable back sheet on a lower side, and an absorber provided between the top sheet and the back sheet. Side portions of the main body are individually provided with a side sheet, and are further provided with a pair of wings extending outward from the main body in a width direction. The wing is formed mainly by bonding a part of the back sheet and a part of the side sheet. In a state in which the wing is folded to the lower side when the article is worn, the side sheet wraps around a folding line formed at the time of folding to form a flexible portion, and an outer end of the flexible portion in the width direction is positioned outside the folding line in the width direction.

## Description

### [Technical Field]

The present invention relates to an absorbent article.

### [Background Art]

As absorbent articles, sanitary napkins, incontinence pads, panty liners and the like are known. Typically, such an absorbent article includes a main body having a top sheet on an upper side (a side facing the skin), a back sheet on a lower side (a side facing underwear), and an absorber provided between the two sheets. Further, in many cases, a pair of side sheets spaced apart from each other are disposed on both side portions of the main body in the front-rear direction mainly for preventing leakage of body fluid from the sides (for example, Patent Document 1). The pair of side sheets is generally made of a nonwoven fabric or the like.

### [Related Art Document]

### [Patent Document]

[Patent Document 1] Japanese Patent Application Laid-Open No. 2005-312694

### [Summary of the Invention]

### [Problem to be Solved by the Invention]

In a state where the absorbent article is worn, the side portions of the main body may contact with respective inner leg sides of the wearer. Since the side sheets provided on both side portions are often made of a nonwoven fabric or the like as described above, discomfort on the inner sides of legs can be reduced by the softness of the material. However, if the wearer moves the legs back and forth, for example when walking or running, the end portions of the main body may contact and rub against the inner sides of the legs. In that case, a wearer with weak skin may feel discomfort or pain. Therefore, there is a demand for an absorbent article in which even a wearer with weak skin feels less uncomfortable or the like that may occur due to contact with the side portions of the main body.

An object of one aspect of the present disclosure is to provide an absorbent article that reduces discomfort or the like that may occur due to contact with side portions of the main body.

### [Means for Solving the Problem]

According to one aspect of the present disclosure, an absorbent article includes a main body having a permeable top sheet on an upper side, an impermeable back sheet on a lower side, and an absorber provided between the top sheet and the back sheet, wherein side portions of the main body are individually provided with a side sheet, wherein a pair of wings extending outward from the main body in a width direction are further provided, wherein the wing is formed mainly by bonding a part of the back sheet and a part of the side sheet, wherein, in a state in which the wing is folded to the lower side when the article is worn, the side sheet wraps around a folding line formed at the time of folding to form a flexible portion having a ring-shaped cross-section at any position including both end positions thereof in a front-rear direction, and wherein an outer end of the flexible portion in the width direction is positioned outside the folding line in the width direction.

### [Effects of the Invention]

According to one aspect of the present disclosure, it is possible to provide an absorbent article in which discomfort or the like that may occur due to contact with the side portions of the main body is reduced.

### [Brief Description of the Drawings]

[FIG. 1] FIG. 1 is a plan view of an absorbent article according to a first embodiment of the present disclosure.
[FIG. 2] FIG. 2 is a cross-sectional view taken along line I-I of FIG. 1.
[FIG. 3] FIG. 3 is an enlarged view of a portion where a side sheet is disposed in FIG. 2.
[FIG. 4] FIG. 4 is a plan view showing a state in which the absorbent article shown in FIG. 1 is worn.
[FIG. 5] FIG. 5 is a cross-sectional view taken along line II-II of FIG. 4.
[FIG. 6] FIG. 6 is a view for explaining formation of a flexible portion.
[FIG. 7] FIG. 7 is a plan view of an absorbent article according to a second embodiment of the present disclosure.
[FIG. 8] FIG. 8 is a cross-sectional view taken along line III-III of FIG. 7.
[FIG. 9] FIG. 9 is a plan view showing a state in which the absorbent article shown in FIG. 7 is worn.
[FIG. 10] FIG. 10 is a cross-sectional view taken along line IV-IV of FIG. 9.
[FIG. 11] FIG. 11 is a plan view of an absorbent article according to a third embodiment of the present disclosure.
[FIG. 12] FIG. 12 is a cross-sectional view taken along line V-V of FIG. 11.

### [Embodiments for Carrying out the Invention]

Hereinafter, embodiments of the present disclosure will be described with reference to the drawings. In the drawings, the same or corresponding components are denoted by the same reference numerals and the description thereof may be omitted unless otherwise specified.

### <First Embodiment>

### (Basic Structure of Absorbent Article)

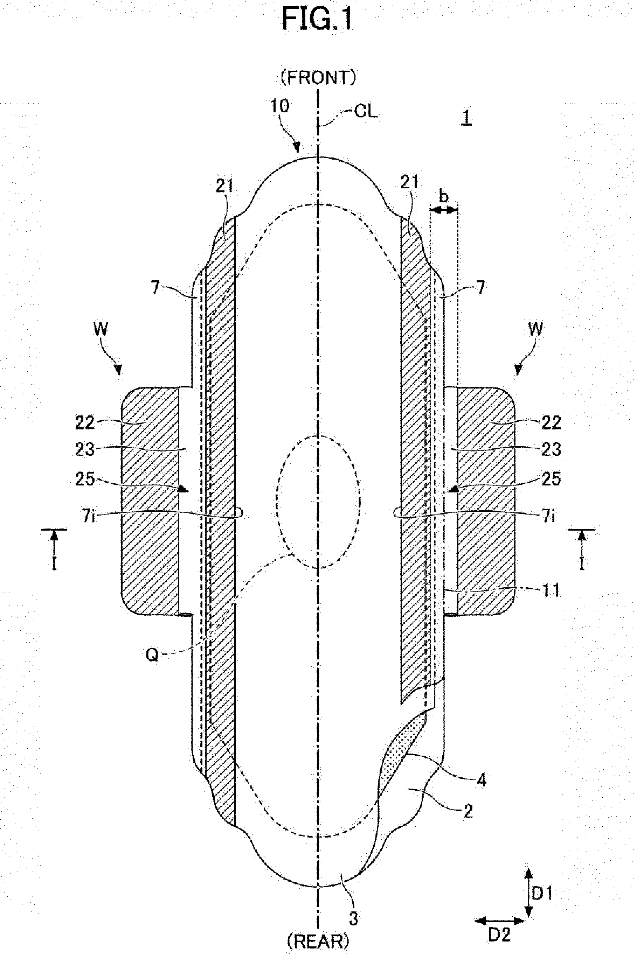

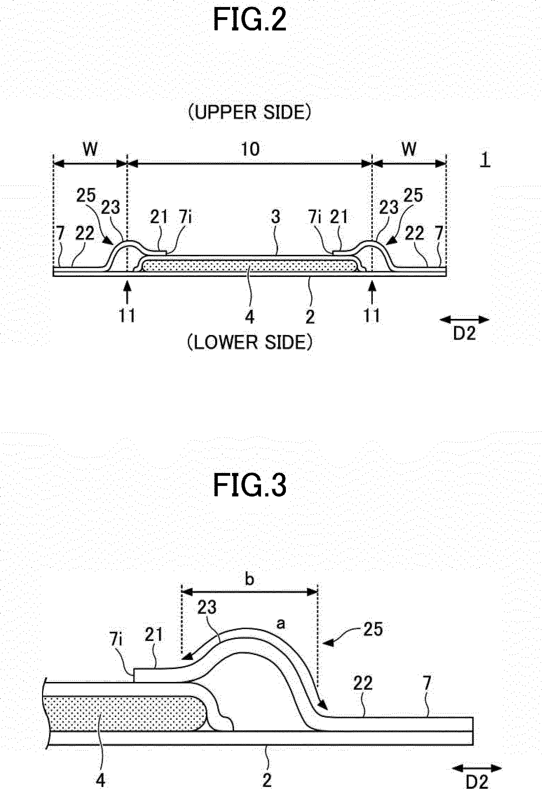

First, a basic structure of an absorbent article according to an embodiment of the present disclosure will be described by taking a sanitary napkin as an example. FIG. 1 shows a plan view of an absorbent article 1 according to a first embodiment. FIG. 2 shows a cross-sectional view taken along line I-I in FIG. 1.

As shown in FIGS. 1 and 2, the absorbent article 1 includes a main body (absorbent article main body) 10 having a permeable top sheet 3 on an upper side, an impermeable back sheet 2 on a lower side, and an absorber 4 disposed between the back sheet 2 and the top sheet 3. As shown in FIG. 2, the main body 10 has a flat shape.

In this specification, the top sheet 3 side of the absorbent article 1 is referred to as "upper side", and the back sheet 2 side is referred to as "lower side". That is, the "upper side" of the absorbent article 1 is the side (skin side or front side) facing the skin when the article is worn, and the "lower side" is the side (underwear side or back side) facing the underwear when the article is worn. The plan view of FIG. 1 is a view of the absorbent article 1 as viewed from above.

Also, in this specification, as shown in FIG. 1, a direction corresponding to the front-rear direction of the body of the wearer when the absorbent article 1 is worn is referred to as a front-rear direction D1 of the absorbent article 1, and a direction orthogonal to the front-rear direction D1 is referred to as a width direction D2 of the absorbent article 1. The main body 10 may have an elongated shape in the front-rear direction D1 in a plan view, may have a predetermined length in the front-rear direction D1, and may have a predetermined width in the width direction D2 orthogonal to the front-rear direction D1.

In the present embodiment shown in FIG. 1, the main body 10 has a shape substantially line-symmetric with respect to a centerline (front-rear direction centerline) CL extending in the front-rear direction D1 in a plan view, however, the main body 10 may not be line-symmetric. In addition, configurations other than the shapes of the absorbent article 1 and the main body 10 (including the thickness and density of the absorber 4, the size and position of a compressed groove, the shape and size of the wing or flap, and the like) may be substantially symmetric or may be asymmetric, with respect to the centerline as a symmetry axis.

The back sheet 2 may be a sheet having at least water shielding properties, and may be, for example, a sheet made of an olefin resin such as polyethylene or polypropylene. It is also possible to use a polyethylene-sheet-laminated nonwoven fabric or a nonwoven fabric layered sheet having a waterproof film interposed therebetween such that impermeability is substantially ensured. Further, a sheet material having moisture permeability is desirably used to prevent stuffiness. As such a water shielding and moisture permeable sheet material, a microporous sheet is preferably used. The microporous sheet is obtained by forming a sheet by melting and kneading an inorganic filler with an olefin resin such as polyethylene or polypropylene, and stretching the sheet in a uniaxial direction or biaxial directions. The back sheet 2 may be a sheet having the same outer shape as that of the absorbent article 1.

The top sheet 3 may be a permeable sheet that allows body fluids such as menstrual blood, vaginal discharge, urine, and the like to promptly permeate therethrough. Examples of suitable materials that may be used as the top sheet 3 include a porous or non-porous nonwoven fabric, a porous plastic sheet, and the like. Examples of material fibers constituting the nonwoven fabric include synthetic fibers such as olefin such as polyethylene or polypropylene, polyester and polyamide; regenerated fibers such as rayon or cuprammonium rayon; mixed fibers thereof; and natural fibers such as cotton. These fibers can be used alone or in combination with two or more kinds. Further, examples of a method for processing a nonwoven fabric include a spunlace method, a spunbond method, a thermal bond method, a melt blown method, and a needle punch method. Among the above methods, the spunlace method is preferred in terms of flexibility, the spunbond method is preferred because a nonwoven fabric with high drape properties can be manufactured, and the thermal bond method is preferred because a soft nonwoven fabric with high bulkiness can be manufactured. Further, composite fibers including core-in-sheath fibers having a high-melting-point fiber as a core and a low-melting-point fiber as a sheath, side-by-side fibers, split fibers, and the like may be used, for example.

The absorber 4 is not particularly limited as long as it is a material capable of absorbing and retaining body fluids, but preferably includes cotton-like pulp and a water-absorbing polymer. Examples of the water-absorbing polymer that may be used include a superabsorbent polymer (SAP), a superabsorbent polymer fiber (SAF), and a combination thereof. Examples of the pulp include cellulose fibers such as dissolving pulp, chemical pulp, and the like obtained from wood, and artificial cellulose fibers such as rayon, acetate, and the like. Softwood materials, hardwood materials, and the like may be used as the raw material for the chemical pulp, however, softwood materials are preferably used in view of their long fiber length and the like.

Also, a synthetic fiber may be mixed into the absorber 4. Examples of the synthetic fiber that may be used include polyolefins such as polyethylene, polypropylene, and the like; polyesters such as polyethylene terephthalate, polybutylene terephthalate, and the like; polyamide such as nylon, and the like; and copolymers thereof. A combination of two or more of these materials may also be used. Further, composite fibers including core-in-sheath fibers having a high-melting-point fiber as a core and a low-melting-point fiber as a sheath, side-by-side fibers, split fibers, and the like may be used, for example. Also, hydrophobic fiber that has been surface-treated with a hydrophilizing agent to exhibit affinity to body fluids may be used, for example. The absorber 4 is preferably manufactured by a fiber stacking method or an air laid method.

In order to prevent twisting or splitting of the absorber, spilling of polymer particles, or the like, the absorber 4 may be surrounded by a colored or an uncolored (white) encapsulating sheet made of a crepe paper or a nonwoven fabric. The thickness of the absorber is preferably 0.5 to 25 mm. The absorber 4 does not have to have a uniform thickness across its entire surface. For example, the absorber 4 may have a structure in which the region including the region corresponding to the body fluid discharge part and the vicinity thereof is bulged.

The absorber 4 may have a shape and a size that do not protrude from the back sheet 2 and the top sheet 3 at least in the front-rear direction D1 in a plan view. At both end portions of the absorber 4 in the front-rear direction D1, end portions of the back sheet 2 and the top sheet 3 may be bonded to each other with an adhesive, heat seal, or the like. Further, it is preferable that the entire absorber 4 has a shape and a size such that the absorber 4 does not protrude from the back sheet 2 in a plan view. The top sheet 3 may not cover the absorber 4 in the width direction D2, but is preferably formed and arranged to cover the entire absorber 4.

### (Wings and Side Sheets)

As shown in FIGS. 1 and 2, the absorbent article 1 may include a pair of wings W and W each extending from the side of the main body 10. An adhesive portion (not shown) may be disposed on each of the lower side (back sheet side) of the wings W and W. When the absorbent article 1 is worn, the wings W and W are folded downward to the lower side (to the back side of the underwear), and the adhesive portions secure the wings to the underwear, thereby preventing the entire absorbent article 1 from shifting or twisting. The position of the pair of wings W and W of the main body in the front-rear direction D1 may be the region including a region corresponding to a body fluid discharge part Q which faces the body fluid discharge part of the wearer when the absorbent article is worn, and a region extending in the front-back direction thereof. In other words, when viewed in the front-rear direction D1, the region corresponding to the body fluid discharge part Q may be disposed in a range from the front to rear ends of the wings W at boundary lines 11 each provided between the main body 10 and the respective wing W.

In the present embodiment, on both side portions of the main body 10, that is, on the upper side (top sheet 3 side) of both end portions in the width direction D2, a pair of side sheets 7 and 7 are disposed over the front-rear direction D1. The side sheet is preferably made of a nonwoven fabric. The nonwoven fabric used for the side sheet may be subjected to a water-repellent treatment or a hydrophilic treatment, and a water-repellent nonwoven fabric is preferable. In the water-repellent treatment, a coating agent such as a silicone-based or a paraffin-based water-repellent agent may be used. The water-repellent nonwoven fabric is used to prevent the permeation of body fluids such as menstrual blood, vaginal discharge, urine, and the like. The type of the nonwoven fabric is not particularly limited, and may be a nonwoven fabric processed by an air-through method, a spunlace method, a spunbond method, a melt blown method, or a needle punch method. However, in order to improve the function of the flexible portion (which will be described in detail later), a soft air-through nonwoven fabric that is less likely to be creased and less likely to be wrinkled is preferable. The type of fibers constituting the nonwoven fabric is also not particularly limited, and examples of material fibers include synthetic fibers such as olefin such as polyethylene or polypropylene, polyester and polyamide; regenerated fibers such as rayon or cuprammonium rayon; mixed fibers thereof; and natural fibers such as cotton. These fibers can be used alone or in combination with two or more kinds.

As shown in FIGS. 1 and 2, portions of the side sheets 7 and 7 extend outward in the width direction D2, and are bonded to the portions of the back sheet 2 also extending in the width direction D2 to form the pair of wings W and W. The side sheet 7 and the back sheet 2 may be bonded to each other with an adhesive, a heat seal, an ultrasonic seal, or the like. For the purpose of enhancing the bonding strength and stiffness, dot-shaped or differently shaped compressed portions (embosses) may be formed on the outer end portions of the wings W and W.

In the example shown in FIGS. 1 and 2, each of the side sheets 7 and 7 is constituted by one sheet, but the side sheet 7 may be formed by stacking two or more sheets of the nonwoven fabric as described above.

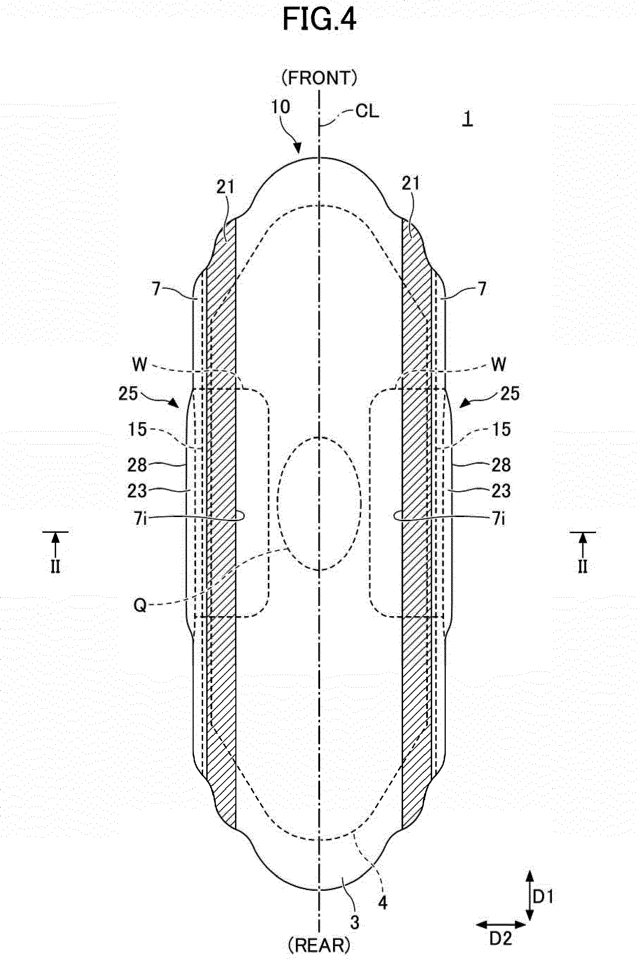

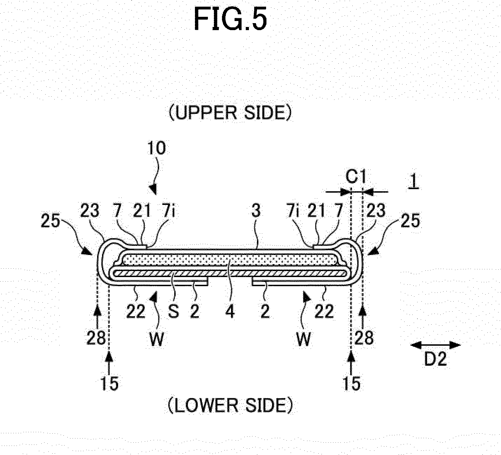

FIG. 4 shows a plan view of a state in which the absorbent article 1 is worn on underwear (not shown). FIG. 5 shows a cross-sectional view taken along line II-II in FIG. 4. As shown in FIG. 5, the wings W and W can be folded downward to sandwich underwear S between the main body 10 and the wings W. A folding line (folding line along which the back sheet is folded) 15 formed at the time of folding the wing does not necessarily need to be along the boundary line 11 between the main body 10 and the wing W, and may be located, for example, on the inner side of the boundary line 11 (FIG. 2) in the width direction D2. Alternatively, it may be located on the outer side of the boundary line 11 in the width direction D2.

### (Flexible Portion)

In the present embodiment, the side sheet 7 is not entirely bonded to a component (such as the back sheet 2) disposed on the lower side thereof, and has a region not partially bonded to anywhere. That is, the side sheet 7 may have a bonded portion that is bonded to the component disposed on the lower side thereof and a non-bonded portion that is not bonded. In the example of FIGS. 1 and 2, the side sheet 7 may have an inner bonded portion 21 bonded to the top sheet 3 of the main body 10, an outer bonded portion 22 bonded to the back sheet 2 in the wing W, and a non-bonded portion 23 located between the inner bonded portion 21 and the outer bonded portion 22 and not bonded to the main body 10 and/or the back sheet 2. The non-bonded portion 23 of the side sheet 7 is placed in a sagging position so that the portion bulges upward, thus forming a flexible portion 25 having a hollow therein.

FIG. 3 shows a partially enlarged view of FIG. 2 (an enlarged view of a portion where the side sheet 7 is disposed). The above-mentioned bulge (sag) of the non-bonded portion 23 of the side sheet 7, that is the flexible portion 25, is formed by making a length a of the non-bonded portion 23 of the side sheet 7 in the width direction D2 (the length of the non-bonded portion of the side sheet 7 in the width direction D2) longer than a distance b between an outer end of the inner bonded portion 21 in the width direction D2 and an inner end of the outer bonded portion 22 in the width direction D2.

As shown in FIGS. 4 and 5, when the pair of wings W and W are folded to the back side of the underwear S at the time of wearing, each of the flexible portions 25 and 25 formed on the both sides of the front-rear direction centerline CL may protrude outward in the width direction D2. To be more specific, the flexible portions 25 and 25 are formed beyond the folding lines 15 and 15 of the wings W and W outward in the width direction D2. That is, in a state in which the wings W and W are folded downward, outer ends 28 and 28 of the respective flexible portions 25 and 25 in the width direction D2 are located on the outer sides of the folding lines 15 and 15 of the respective wings W and W in the width direction D2. The flexible portions 25 and 25 are hollow and, as shown in FIG. 5, have a ring-shaped cross-section taken along the width direction D2. In the present specification, the term "annular" refers to a shape of a curved line that can be formed by bending a straight line once, and may be a partial circle or a partial ellipse.

The ring shape of the flexible portion 25 may be formed not only with the non-bonded portion 23 of the side sheet 7 substantially alone but also with the non-bonded portion 23 and the top sheet 3 and/or the back sheet 2. In either case, the non-bonded portion 23 of the side sheet 7 comes into contact with the leg portion of the wearer. In addition, the ring shape includes a so-called loop shape (hollow inside), and a case where a hollow is not substantially formed inside (the hollow is collapsed) before wearing but the hollow can be formed when worn. However, as will be described later in detail, the flexible portion 25 in the present embodiment has a hollow shape in any cross-section, and is not bonded so as not to have a hollow shape in the end portions or the central portion thereof, for example.

In this way, with the wings W and W folded downward, the folding lines 15 and 15 of the respective wings W and W are wrapped by the side sheets 7 and 7, and the flexible portions 25 and 25 protrude outward in the width direction D2. Therefore, when the article is worn, the flexible portions 25 and 25 come into contact with the inner sides of the wearer's groin, and the folding lines 15 and 15 do not come into direct contact with the inner sides of the wearer's groin. Since the flexible portions 25 and 25 are hollow and have elasticity, the wearer may feel softness and less discomfort when wearing. In the conventional form without the flexible portions 25 and 25, the folding lines 15 and 15 of the respective wings W and W are in direct contact with sides of the groin, and high stiffness of the folding lines 15 and 15 and their adjacent portions tend to cause a sense of discomfort to the delicate skin on the inner sides of the groin. However, according to the present embodiment, the legs come into direct contact with the flexible portions 25 and 25, and contact between the wearer's legs and the folding lines 15 and 15 can be avoided, so that a comfortable wearing feeling can be provided.

In addition, even when the wearer is walking or running, that is, when the wearer's legs are moved back and forth, the flexible portions 25 and 25, which are elastic and soft, can be deformed with respect to the force from the legs and can follow the force to some extent, therefore discomfort or pain due to rubbing is less likely to occur.

Further, the flexible portions 25 and 25 have a ring-shaped cross-section at any position including both end positions thereof in the front-rear direction D1. That is, the flexible portions 25 and 25 have a ring-shaped cross-section not only at the center in the front-rear direction D1 but also at both end portions in the front-rear direction D1. In other words, the flexible portions 25 and 25 have a ring-shaped cross-section that is continuous over the front-rear direction D1, and have a so-called cylindrical shape. The ring shape of the flexible portions 25 and 25 may vary in the front-rear direction D1, that is, the degree of curvature or the size of the ring shape may change at some points, however, the hollow of the flexible portions 25 and 25 is maintained over the front-rear direction D1. This means that the flexible portions 25 and 25 do not have any portion which is crushed or where the inner surface of the flexible portions 25 and 25 is bonded therein. This is also apparent from the fact that the non-bonded portions 23 and 23 of the side sheets 7 and 7 extend continuously in the front-rear direction as shown in FIGS. 1 and 4.

For example, in a case where the flexible portions 25 and 25 are crushed at the front and rear end portions thereof and are fixed in the crushed state, the fixed state affects even the center of the flexible portions 25 and 25 in the front-rear direction D1. Then, it becomes difficult for the flexible portions 25 and 25 to move flexibly, and there is a possibility that the flexibility of the flexible portions 25 and 25 is impaired. On the other hand, in the present embodiment in which the flexible portions 25 and 25 have a ring-shaped cross-section at any position including both end positions thereof in the front-rear direction D1, the elasticity and softness of the flexible portions are maintained over the entire front-rear direction D1 of the flexible portions 25 and 25. Therefore, even when the wearer's legs are greatly moved back and forth, the function of the flexible portions 25 and 25 is exhibited over the front-rear direction D1, and the uncomfortable feeling or the like due to rubbing is reduced.

In a state where the wings W and W are folded downward, a length C1 (FIG. 5) from the folding lines (folding positions) 15 and 15 of the respective wings W and W to the outer ends 28 and 28 of the flexible portions 25 and 25 in the width direction D2 may be about 1 to 10 mm. The length C1 is a length measured in a natural state in which the absorbent article 1 is worn on the underwear S.

Although the length C1 depends on the thickness or stiffness of the side sheet 7 used for the flexible portion 25, the size of the flexible portion 25 can be adjusted by the relationship between the length a of the non-bonded portion 23 of the side sheet 7 used in the width direction D2 and the distance b between the inner bonded portion 21 and the outer bonded portion 22 in the width direction D2 (FIG. 3). In order to obtain an appropriate flexible portion 25, for example, a difference (a - b) between the length a of the non-bonded portion 23 in the width direction D2 and the distance b between the inner bonded portion 21 and the outer bonded portion 22 in the width direction D2 can be preferably set to 5 to 10 mm in the state where the wing W is spread.

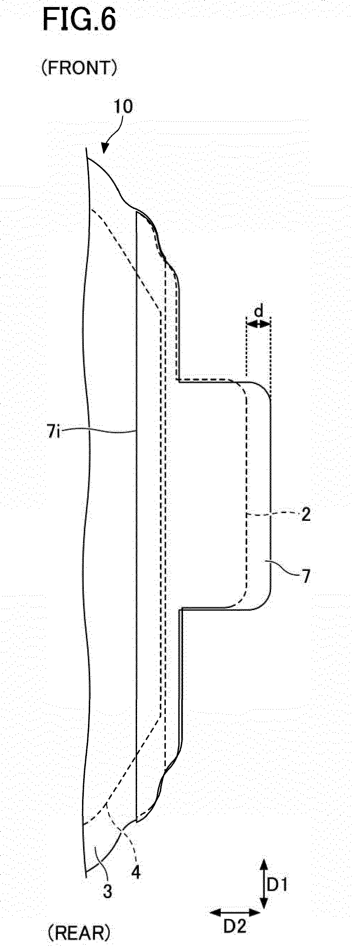

For this purpose, the side sheets 7 and 7 used in the present embodiment are longer in length in the width direction D2 than the side sheets for producing a conventional configuration without the flexible portions 25 and 25. FIG. 6 shows a plan view of a state in which the flat side sheet 7 is superposed on the main body in the production stage of the absorbent article 1. As shown in FIG. 6, the side sheet 7 longer than the back sheet 2 constituting a part of the wing by a length d in the width direction D2 is used. While sagging the non-bonded portion 23 of the side sheet 7, the inner bonded portion 21 and the outer bonded portion 22 are bonded to the component of the main body 10 disposed therebelow (FIGS. 2 and 3) . The length d can be equal to the difference (a - b) between the length a of the non-bonded portion 23 in the width direction D2 and the distance b between the inner bonded portion 21 and the outer bonded portion 22.

The length a of the non-bonded portion 23 in the width direction D2 (FIG. 3) may be 5 to 20 mm. By setting the length a in this range, an appropriate size of the flexible portion 25 can be secured, the flexible portion 25 can follow the movement of the legs to some extent even when the legs are moved back and forth, and a sense of discomfort caused by the flexible portion 25 becoming excessively large can be prevented. Further, the distance b between the inner bonded portion 21 and the outer bonded portion 22 in the width direction D2 may be 3 to 15 mm. By setting the distance b in this range, the flexible portion 25 having a flexible ring cross-section can be formed.

The position where the flexible portion 25 is provided in the width direction D2 (the position where the non-bonded portion 23 is formed) is not limited as long as the flexible portion 25 can protrude outward in the width direction D2 when worn, however, the position is preferably outward from the absorber 4 in the width direction D2, in a plan view, and more preferably outward from the absorber 4 by greater than or equal to 2 mm in the width direction D2.

Each of the side sheets 7 may be a single sheet extending in the planar direction, but may be folded at the end portion in the width direction D2, for example. However, as shown in FIGS. 1 to 5, for example, an inner end portion 7i of the side sheet 7 in the width direction D2 is not folded to the lower side or to the upper side, but is directed inward in the width direction D2. In such a simple configuration without folding as shown in the drawings, the configuration of the flexible portion 25 is simplified, and it is possible to prevent loss of flexibility due to folding and stacking of sheets or the like. Further, for example, in the case where the inner end portion 7i of the side sheet 7 is folded downward to the lower side in the width direction D2, and the surface of the side sheet 7 facing the upper side is bonded to the underlying component before the folding downward, when the wings W and W are folded back at the time of wearing, the flexible portion 25 is likely to protrude upward and is unlikely to face outward in the width direction D2. However, according to the present embodiment, the flexible portion 25 naturally protrudes outward in the width direction D2 to wrap around the folding line 15.

It is preferable that the flexible portion 25 does not include a stretchable member such as a rubber thread or a stretchable sheet. By forming the flexible portion 25 with a sheet such as a nonwoven fabric, it is possible to prevent a sense of discomfort or the like when the portion touches the skin.

### <Second Embodiment>

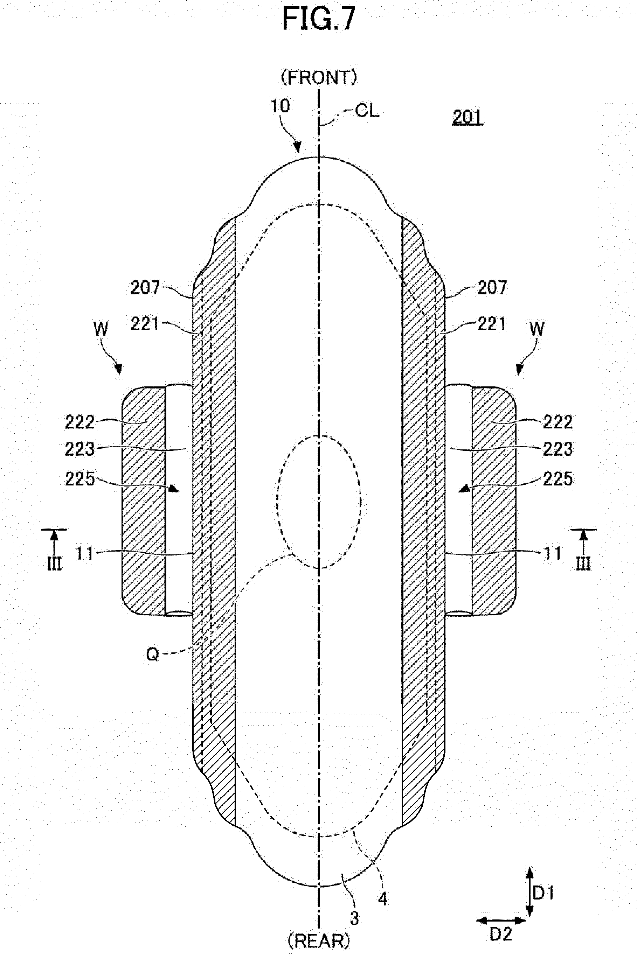

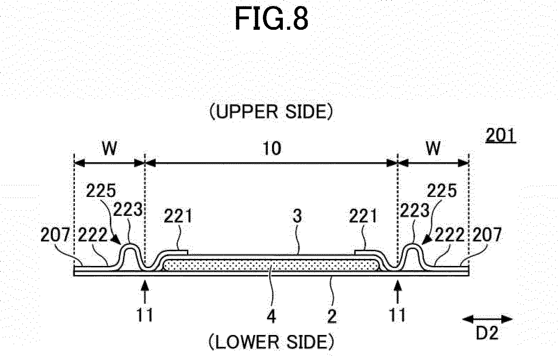

FIG. 7 shows a plan view of an absorbent article 201 according to a second embodiment. FIG. 8 shows a cross-sectional view taken along line III-III in FIG. 7. The basic configuration and the obtained basic effects of the second embodiment are the same as those of the first embodiment. Also, in the absorbent article 201, side sheets 207 and 207 are disposed on both side portions of the main body 10. An inner bonded portion 221 and an outer bonded portion 222 of each side sheet 207 are bonded to the underlying component, while a non-bonded portion 223 is formed between the inner bonded portion 221 and the outer bonded portion 222. The non-bonded portion 223 is raised from the underlying component (back sheet 2), thereby forming a flexible portion 225. In the second embodiment, however, the positions of the flexible portions 225 and 225 are different from those in the first embodiment.

As shown in FIGS. 7 and 8, the flexible portions 225 and 225 of the second embodiment are disposed slightly outward in the width direction D2 compared to the positions of the flexible portions 25 and 25 in the first embodiment. More specifically, each of the flexible portions 225 and 225 shown in FIGS. 7 and 8 is formed adjacent to the boundary line 11 between the body 10 and the wing W. However, the flexible portion 225 may be formed at least within the region of the wing W, and may be formed at a position beyond the boundary line 11. In other words, in the present embodiment, the non-bonded portion 223 of the side sheet 207 may be formed in the wing W region. Alternatively, in a condition that the outer end of the inner bonded portion 221 of the side sheet 207 in the width direction D2 does not cross the boundary line 11 between the main body 10 and the wings W, the inner end of the outer bonded portion 222 of the side sheet 207 in the width direction D2 may be located within the wings W region.

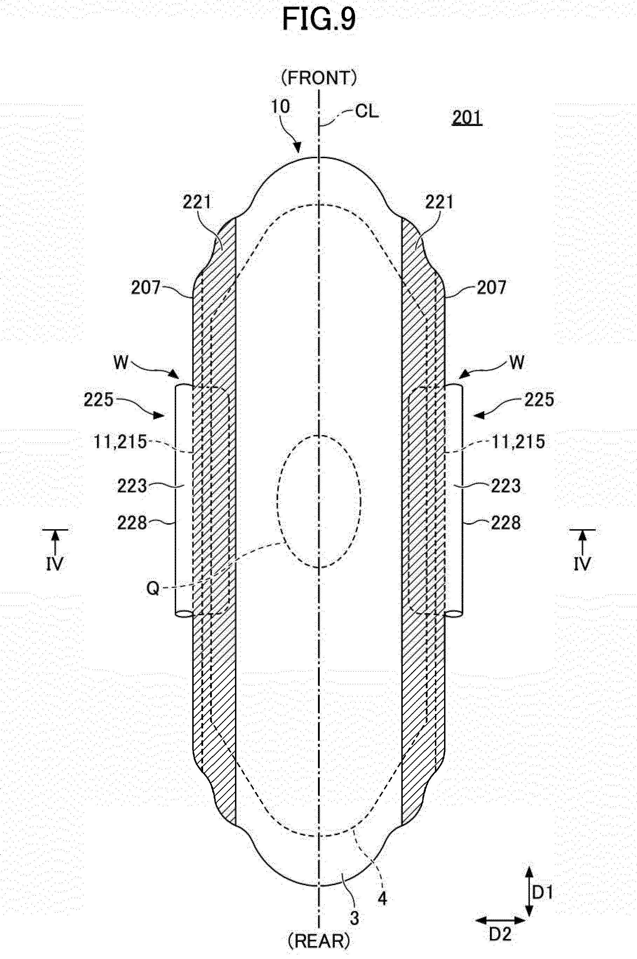

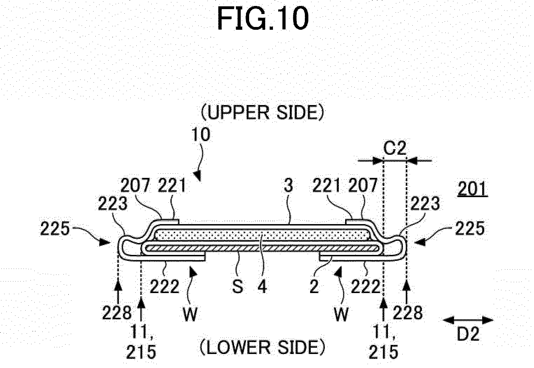

Further, FIG. 9 shows a plan view of the absorbent article 201 in a state where the pair of wings W and W are folded when the article is worn, and FIG. 10 shows a cross-sectional view taken along line IV-IV of FIG. 9. In the present embodiment shown in FIGS. 9 and 10, unlike the folding method shown in FIGS. 4 and 5, each of the wings W and W is folded at the boundary lines 11 and 11 between the main body 10 and the wings W and W. That is, according to the folding method of the wings W and W in the present embodiment, folding lines 215 and 215 overlap the boundary lines 11 and 11, respectively. However, the position where the folding line 215 is formed may be outside the boundary line 11 in the width direction D2, or may be inside the boundary line 11 in the width direction D2 as in the folding method exemplified in the first embodiment (FIGS. 4 and 5).

According to the second embodiment shown in FIGS. 7 to 10, since the positions of the flexible portions 225 and 225 are disposed further outward in the width direction D2 than in the first embodiment, when the pair of wings W and W are folded to the lower side of the underwear S, the tendency of the flexible portions 225 and 225 to rise upward is suppressed, and the flexible portions can be formed to protrude further outward in the width direction D2. Therefore, it is possible to more reliably exhibit the function of the flexible portions 225 and 225, such as suppressing a sense of discomfort or the like that may be caused often when the main body 10 of the absorbent article 1 in the width direction D2 is in contact with the inner sides of the legs.

Also, in the second embodiment, in a state where the wings W and W are folded, a length C2 from the folding lines (folding positions) 215 and 215 of the respective wings W and W to outer ends 228 and 228 of the flexible portions 225 and 225 in the width direction D2 (FIG. 10) may be about 1 to 10 mm.

### <Third Embodiment>

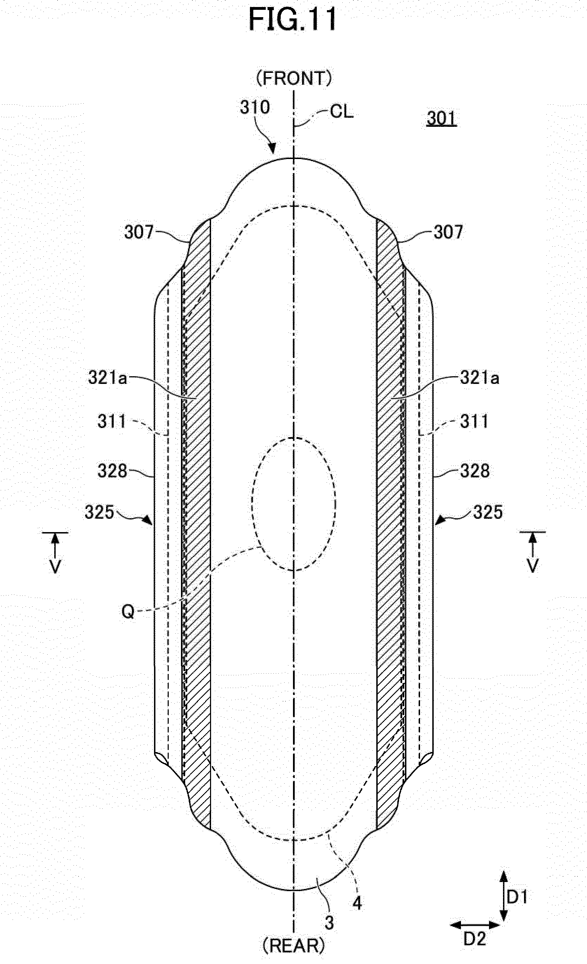

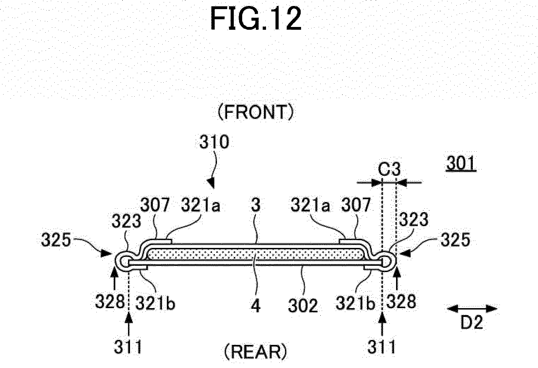

FIG. 11 shows a plan view of an absorbent article 301 according to a third embodiment of the present disclosure. FIG. 12 shows a cross-sectional view taken along line V-V in FIG. 11. The basic configuration of the absorbent article 301 and the obtained basic effects of the third embodiment are like those of the first embodiment (FIGS. 1 to 5) and the second embodiment (FIGS. 7 to 10). Also, in the absorbent article 301, side sheets 307 and 307 are disposed on both side portions of a main body 310.

The third embodiment differs from the first and second embodiments in that there are no wings. Without wings, a back sheet 302 has substantially the same elongated shape in a plan view as the main body 310 without lateral extensions. Further, the side sheets 307 and 307 have a shape without any extending portion in the width direction in a plan view.

The side sheets 307 and 307 are bonded to wrap end portions 311 and 311 of the main body 310 in the width direction D2 (outer ends in the width direction D2), respectively. To be more specific, as shown in FIG. 11, a bonded portion 321a of the side sheet 307 is bonded to a component on the upper side (the top sheet 3 in the present embodiment) of the main body 310, and a bonded portion 321b is bonded to a component in the lower side (the back sheet 2 in the present embodiment) of the main body 310. Between the bonded portion 321a and the bonded portion 321b of the side sheet 307, a non-bonded portion 323 not bonded to the other component is formed. The non-bonded portion 323 of the side sheet 307 is placed in a sagging position so that the portion bulges upward, thus forming a flexible portion 325 having a hollow therein.

As shown in FIGS. 11 and 12, the flexible portion 325 protrudes outward from the end portion 311 of the main body 310 in the width direction D2. For this reason, it is not the end portion 311 of the main body 310 in the width direction D2 but the flexible portion 325 that comes into direct contact with the skin on the inner side of the leg when the article is worn. A length C3 from the end portion 311 of the main body 310 to an outer end 328 of the flexible portion 325 in the width direction D2 (FIG. 11) may be about 1 to 10 mm.

The flexible portion 325 has a ring-shaped cross-section when viewed in the width direction D2. In the illustrated embodiment, the ring-shaped cross-section is formed with the flexible portion 325 and a portion of the back sheet 302, but the flexible portion 325 alone may form the ring shape. The flexible portion 325 has higher elasticity and flexibility than other portions of the absorbent article 1, and when both side portions of the absorbent article 1 come into contact with the wearer's skin on the inner sides of the groin of the legs, there is less discomfort, and even when the wearer rubs the side portions against the inner sides of the legs during walking or the like, discomfort or pain is less likely to occur. In addition, in this embodiment, the ring-shaped cross-section of the flexible portion 325 is maintained at any position including both end positions in the front-rear direction D1. The elasticity and flexibility of the flexible portion 325 are maintained over the front-rear direction D1, and even if the wearing position of the absorbent article 1 on the underwear is slightly shifted in the front-back direction, the function of the flexible portion 325 can be exhibited.

Hereinafter, specific embodiments of the present disclosure will be described.

### (Appended Clause 1)

An embodiment according to Appended Clause 1 provides an absorbent article including a main body having a permeable top sheet on an upper side, an impermeable back sheet on a lower side, and an absorber provided between the top sheet and the back sheet, wherein side portions of the main body are individually provided with a side sheet, and are further provided with a pair of wings extending outward from the main body in a width direction, wherein the wing is formed mainly by bonding a part of the back sheet and a part of the side sheet, wherein in a state in which the wing is folded to the lower side when the article is worn, the side sheet wraps around a folding line formed at the time of folding to form a flexible portion having a ring-shaped cross-section at any position including both end positions thereof in a front-rear direction, and wherein the flexible portion has an outer end in the width direction which is positioned outside the folding line in the width direction.

In the embodiment according to Appended Clause 1, in a state in which the wing is folded, the side sheet wraps around the folding line of the wing to form a flexible portion having a ring-shaped cross-section. The outer end of the flexible portion in the width direction is located outside of the folding line of the wing. Thus, during wearing of the absorbent article, the inner sides of the wearer's legs are not in direct contact with the folding lines of the wings, but with the flexible portions formed by the side sheets. Since the flexible portions are hollow with a ring-shaped cross-section, elastic and flexible, the wearer feels better when the flexible portions come into contact with the inner sides of the legs than when the folding lines of the wings with no flexibility come into direct contact therewith. In addition, even when the legs are moved back and forth, since the flexible portions can follow the movement of the legs to some extent, discomfort or pain due to rubbing is less likely to occur.

Further, the flexible portion is configured to have a ring-shaped cross-section at any position including both end positions thereof in the front-rear direction. That is, the flexible portion maintains a ring-shaped cross-section even at both end portions in the front-rear direction, and the flexible portion is not crushed at front of and/or back portions thereof. Therefore, compared to a configuration in which the inner surface of the flexible portion is bonded at a front and/or back portion thereof, elasticity and flexibility are maintained over the entire front-rear direction of the flexible portion. Therefore, even when the legs are largely moved in the front-rear direction, an effect of reducing a feeling of strangeness or the like due to rubbing can be obtained.

### (Appended Clause 2)

In an embodiment according to Appended Clause 2, the side sheet includes an inner bonded portion bonded to the main body at the inner side in the width direction, an outer bonded portion bonded to the back sheet at the outer side in the width direction, and a non-bonded portion located between the inner bonded portion and the outer bonded portion, and a length of the non-bonded portion in the width direction is 5 to 10 mm longer than a distance between the outer end of the inner bonded portion and the inner end of the outer bonded portion in the width direction, in a state where the pair of wings are unfolded.

In the embodiment according to Appended Clause 2, it is possible to form a flexible portion having an appropriate size. Sufficient elasticity and flexibility can be imparted to the flexible portion, and the flexible portion itself can be prevented from becoming too large to cause discomfort.

### (Appended Clause 3)

In an embodiment according to Appended Clause 3, the flexible portion is formed within the wing.

In the embodiment according to Appended Clause 3, since the flexible portions are not formed on the main body but on the outer side of the main body (outward in the width direction), the flexible portions can more reliably protrude in the width direction at the time of wearing. Therefore, it is possible to more reliably exhibit the function of the flexible portions, such as reducing discomfort or the like that may occur due to contact with the inner sides of the legs.

### (Appended Clause 4)

An embodiment according to Appended Clause 4 provides an absorbent article including a main body having a permeable top sheet on an upper side, an impermeable back sheet on a lower side, and an absorber provided between the top sheet and the back sheet, wherein side portions of the main body are individually provided with a side sheet, wherein the side sheet is bonded to the lower side and the upper side of the main body to wrap an outer end of the main body in a width direction, so that a flexible portion having a ring-shaped cross-section at any position including both end positions thereof in a front-rear direction is formed, and wherein the flexible portion has an outer end positioned outside of the outer end of the main body in the width direction.

In the embodiment according to Appended Clause 4, it is possible to provide a wingless absorbent article that achieves the same effect as the embodiment according to Appended Clause 1.

### (Appended Clause 5)

In an embodiment according to Appended Clause 5, an inner end portion of the side sheet in the width direction is not folded at least downward.

In the embodiment according to Appended Clause 5, since the inner end portion of the side sheet in the width direction is not folded to the lower side, the flexible portion has a stronger tendency to protrude laterally (outward in the width direction) than to rise upward. Therefore, the elasticity and flexibility of the flexible portion can be sufficiently exhibited at the side portion of the main body.

This application claims priority to Japanese Patent Application No. 2021-057751 filed on March 30, 2021 with the Japan Patent Office, the entire contents of which are incorporated herein by reference.

### [List of Reference Numerals]

- 1, 201, 301: Absorbent article
- 2, 302: Back sheet
- 3: Top sheet
- 4: Absorber
- 7, 207, 307: Side sheet
- 10, 310: Main body (absorbent article main body)
- 11, 311: End portion of the main body in the width direction
- 15, 215: Folding line
- 21, 221: Inner bonded portion
- 22, 222: Outer bonded portion
- 23, 223, 323: Non-bonded portion
- 25, 225, 325: Flexible portion
- 28, 228, 328: Outer end of the flexible portion in the width direction
- 321a, 321b: Bonded portion
- CL: Front-rear direction centerline
- D1: Front-rear direction
- D2: Width direction
- Q: Region corresponding to body fluid discharge part
- W: Wing

## Claims

1. An absorbent article comprising a main body having a permeable top sheet on an upper side, an impermeable back sheet on a lower side, and an absorber provided between the top sheet and the back sheet,
wherein side portions of the main body are individually provided with a side sheet,
wherein a pair of wings extending outward from the main body in a width direction are further provided,
wherein the wing is formed mainly by bonding a part of the back sheet and a part of the side sheet,
wherein in a state in which the wing is folded to the lower side when the article is worn, the side sheet wraps around a folding line formed at a time of folding to form a flexible portion having a ring-shaped cross-section at any position including both end positions thereof in a front-rear direction, and
wherein the flexible portion has an outer end in the width direction which is positioned outside the folding line in the width direction.

2. The absorbent article according to claim 1,
wherein the side sheet includes an inner bonded portion bonded to the main body at an inner side in the width direction, an outer bonded portion bonded to the back sheet at an outer side in the width direction, and a non-bonded portion located between the inner bonded portion and the outer bonded portion, and
wherein a length of the non-bonded portion in the width direction is 5 to 10 mm longer than a distance between an outer end of the inner bonded portion in the width direction and an inner end of the outer bonded portion in the width direction, in a state where the pair of wings are unfolded.

3. The absorbent article according to claim 1 or 2, wherein the flexible portion is formed within the wing.

4. An absorbent article including a main body having a permeable top sheet on an upper side, an impermeable back sheet on a lower side, and an absorber provided between the top sheet and the back sheet,
wherein side portions of the main body are individually provided with a side sheet,
wherein the side sheet is bonded to the lower side and the upper side of the main body to wrap around an outer end of the main body in a width direction, so that a flexible portion having a ring-shaped cross-section at any position including both end positions thereof in a front-rear direction is formed, and
wherein the flexible portion has an outer end positioned outside of the outer end of the main body in the width direction.

5. The absorbent article according to any one of claims 1 to 4, wherein an inner end portion of the side sheet in the width direction is not folded backwards at least not downward.
